(19) Europäisches Patentamt
European Patent Office
Office européen des brevets

(11) **EP 3 164 012 B1**

(12) **FASCICULE DE BREVET EUROPEEN**

(45) Date de publication et mention
de la délivrance du brevet:
**31.10.2018 Bulletin 2018/44**

(21) Numéro de dépôt: **15753089.0**

(22) Date de dépôt: **01.07.2015**

(51) Int Cl.:
*A23G 3/34* (2006.01)   *A61K 9/20* (2006.01)
*C07C 31/26* (2006.01)   *C07H 3/06* (2006.01)
*A23G 4/10* (2006.01)   *A23L 5/00* (2016.01)

(86) Numéro de dépôt international:
**PCT/FR2015/051820**

(87) Numéro de publication internationale:
**WO 2016/001589 (07.01.2016 Gazette 2016/01)**

(54) **NOUVELLE COMPOSITION EDULCORANTE**

NEUARTIGE SÜSSSTOFFZUSAMMENSETZUNG

NOVEL SWEETENING COMPOSITION

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **01.07.2014 FR 1456288**

(43) Date de publication de la demande:
**10.05.2017 Bulletin 2017/19**

(73) Titulaire: **Roquette Frères
62136 Lestrem (FR)**

(72) Inventeurs:
• **ORTIZ DE ZARATE, Dominique
F-59660 Merville (FR)**
• **LAGACHE, Sylvie
F-59700 Marcq en Baroeul (FR)**
• **BUSOLIN, André
F-59130 LAMBERSART (FR)**
• **BARRE, Antoine
F-59350 Saint-Andre-lez-Lille (FR)**

(74) Mandataire: **Cabinet Plasseraud
66, rue de la Chaussée d'Antin
75440 Paris Cedex 09 (FR)**

(56) Documents cités:
**EP-A2- 0 347 121   FR-A1- 2 890 315
FR-A1- 2 965 819   US-A- 5 324 751**

• **Metin Çelik: "Pharmaceutical Powder
Compaction Technology, Second Edition" In:
"Pharmaceutical Powder Compaction
Technology, Second Edition", 22 août 2011
(2011-08-22), CRC Press, XP055217719, page 173,
tableau 6**
• **NEZZAL A ET AL: "Polymorphism of sorbitol",
JOURNAL OF CRYSTAL GROWTH, ELSEVIER,
AMSTERDAM, NL, vol. 311, no. 15, 15 juillet 2009
(2009-07-15), pages 3863-3870, XP026337346,
ISSN: 0022-0248, DOI:
10.1016/J.JCRYSGRO.2009.06.003 [extrait le
2009-06-06]**

Remarques:
Le dossier contient des informations techniques
présentées postérieurement au dépôt de la demande
et ne figurant pas dans le présent fascicule.

**Description**

DOMAINE DE L'INVENTION

La présente invention concerne une composition édulcorante pulvérulente comprenant entre 80 et 95% en poids de sorbitol cristallisé, la dite composition présentant notamment une excellence aptitude à l'écoulement.

L'invention concerne également le procédé de production de cette nouvelle composition édulcorante. Enfin, l'invention porte également sur l'utilisation de cette composition édulcorante dans un procédé de préparation de chewing-gums et de comprimés.

ARRIERE-PLAN TECHNOLOGIQUE

Le sorbitol ou glucitol est un polyol (ou sucre-alcool) naturel, au pouvoir sucrant deux fois plus faible que le saccharose. À la différence des oses, sa structure ne renferme aucune fonction cétone ou aldéhyde. Le sorbitol est principalement utilisé comme édulcorant de masse pour remplacer le saccharose. Il est aussi utilisé comme séquestrant, excipient, humectant ou stabilisant, dans les médicaments, les cosmétiques et les aliments. Possédant notamment une grande capacité de rétention d'eau, il est responsable de la consistance moelleuse d'un grand nombre de produits alimentaires. L'eau fixée s'évapore difficilement. Le sorbitol est métabolisé lentement par l'organisme et apporte peu de calorie. Chez l'Homme, le sorbitol est métabolisé de la même façon que le glucose : il apporte la même énergie. Toutefois, son métabolisme étant non insulinodépendant, il n'augmente pas la glycémie. Cette propriété est particulièrement intéressante pour les produits destinés aux diabétiques. En outre, il est non fermentescible par les levures.

Le sorbitol pulvérulent, tout comme les autres polyols pulvérulents tel le xylitol ou le mannitol, est plus particulièrement utilisé comme excipient pharmaceutique, comme édulcorant et agent de texture en industrie alimentaire, et comme support d'additif dans tous types d'industries. Sous forme poudre, le sorbitol est un meilleur excipient que le xylitol et le mannitol, et est largement utilisé dans les comprimés et les chewing-gums, en raison de sa très bonne aptitude à la compression et de son effet rafraîchissant.

Le sorbitol, introduit au niveau industriel à la fin de la Seconde Guerre mondiale par la Demanderesse, est actuellement produit par hydrogénation catalytique du dextrose pur. Le dextrose, ou D-glucose, est lui-même traditionnellement obtenu par cristallisation d'un sirop de glucose, lequel constitue le résultat de l'hydrolyse de l'amidon qui est un polymère de réserve du glucose et qui représente le polysaccharide réservoir d'énergie chez de nombreuses plantes.

D'une manière générale, les polyols proposés sous la forme de produits pulvérulents sont entreposés et distribués dans des emballages doubles associant un sac interne fait d'une matière plastique à un sac en papier Kraft ou à une boîte en carton ondulé ou encore en conteneurs souples dits « big bags » ou enfin en vrac. Le conditionnement du sorbitol poudre actuellement commercialisé fait appel à l'un ou l'autre de ces modes d'emballage.

Malgré ces précautions, les poudres commerciales de sorbitol peuvent avoir tendance à s'agglomérer en gros amas. Cette tendance au mottage sera d'autant plus importante que la poudre de sorbitol sera de fine granulométrie.

En général, pour disposer d'un sorbitol cristallisé de grande résistance en compression, on s'efforce de fabriquer un sorbitol de forme cristalline gamma (les formes alpha et beta sont particulièrement instables) en travaillant une solution sursaturée en sorbitol dont la forme gamma représente au moins 90 %. Cependant, même lorsqu'il est cristallisé sous cette forme gamma la plus stable, le sorbitol pulvérulent obtenu classiquement présente un certain nombre d'inconvénients dont celui d'être très hygroscopique.

Cette hygroscopicité élevée conduit à rendre l'écoulement du sorbitol pulvérulent difficile, voire impossible, dès lors qu'une reprise en eau est intervenue. Son utilisation en compression directe s'en trouve alors limitée, nécessitant par exemple de surmonter de sérieuses difficultés de remplissage des presses pour la fabrication de tablettes ou de comprimés.

Pour éviter ce problème d'écoulement du sorbitol pulvérulent, il a été préconisé de préparer un sorbitol de densité faible et de granulométrie plus grossière, comme décrit dans le brevet FR 1 506 334.

Cependant, il est établi que plus la densité apparente d'un sorbitol pulvérulent est faible, plus celui-ci devient friable, c'est-à-dire sensible à une altération de sa granulométrie par action mécanique. En outre, les temps de dissolution de ce produit pulvérulent de granulométrie grossière sont en général trop longs et donc inadaptés. Enfin, si l'aptitude à l'écoulement est partiellement améliorée par la mise en oeuvre de particules d'une telle granulométrie, le caractère hygroscopique résiduel encore trop élevé rend dans tous les cas l'utilisation de ce sorbitol pulvérulent rédhibitoire lorsqu'il est associé à des ingrédients ou à des additifs très sensibles à l'eau.

Il est également établi que la capacité à fixer des quantités importantes d'additifs est directement fonction de la surface spécifique desdites particules. Les capacités d'absorption du sorbitol pulvérulent sont ainsi d'autant plus importantes que sa surface spécifique est élevée. Cependant, il est connu que la surface spécifique des cristaux denses de sorbitol sous forme gamma du commerce est très faible. Ainsi, pour une taille de particules comprise entre 500 et 1000 $\mu$m, au plus égale à 0,7 m2/g.

Dans le but de préparer un sorbitol pulvérulent présentant une meilleure granulométrie, une bonne aptitude à l'écoulement et satisfaisant aux conditions de compressibilité et de friabilité souhaitées, la demande de brevet FR 2 622 190 décrit un sorbitol poudre renfermant des particules d'un diamètre moyen compris entre 300 et 500 μm. Mais la densité apparente élevée et la surface spécifique relativement faible desdites particules, de l'ordre de 0,9 à 1,2 m$^2$/g, ne sont en fait pas significativement modifiées par le procédé de fabrication mis en oeuvre, de sorte que le sorbitol pulvérulent ainsi obtenu conserve le même facteur d'absorption d'humidité et la même solubilité dans l'eau que la poudre de sorbitol de départ.

EP0347121 décrit une composition édulcorante comprenant entre 65 et 95 % en poids sec de sorbitol et et présentant un diamètre volumique comprise entre 200 et 300 microns. La surface spécifique de la poudre est supérieure à 0.6m2/g.

Le brevet EP 1 008 602 appartenant à la société Demanderesse décrit un nouveau sorbitol pulvérulent ainsi que son procédé de préparation. Ce nouveau sorbitol pulvérulent présente à la fois les avantages, le plus souvent incompatibles, de faible hygroscopicité d'une part et de surface spécifique élevée d'autre part ou de faible densité apparente d'une part et de faible friabilité d'autre part, et ce pour une taille de particules relativement faible. Du point de vue de sa composition chimique, le sorbitol pulvérulent revendiqué est relativement pur, c'est-à-dire qu'il présente une richesse, pureté en sorbitol ou teneur en sorbitol pur élevée, généralement supérieure à 95% et plus particulièrement supérieure à 98% en poids. Ainsi La fabrication de ce sorbitol nécessite l'utilisation de matières premières élevées ce qui impacte considérablement les couts de production de ce produit.

Ainsi pour certaines applications il serait intéressant de disposer d'une composition de sorbitol à faible coût de production.

RESUME DE L'INVENTION

La société Demanderesse est parvenue, au prix de nombreuses recherches, à mettre au point une nouvelle composition pulvérulente de sorbitol à faible coût de production et présentant des propriétés tout particulièrement intéressantes pour diverses applications alimentaires et pharmaceutiques.

Ainsi la présente invention concerne une composition édulcorante caractérisée en ce qu'elle comprend de 80 à 95 % en poids sec de sorbitol pulvérulent cristallisé

- et présente une enthalpie au plus égale à 150 J/g,
- un diamètre moyen volumique compris entre 200 et 350 μm, et
- une surface spécifique, déterminée selon la méthode BET, est inférieure à 0,6 m$^2$/g, de préférence comprise entre 0,15 et 0,4 m$^2$/g, et encore plus préférentiellement entre 0,20 et 0,35 m$^2$/g.

De manière préférée, la composition édulcorante selon l'invention est caractérisée en ce qu'elle comprend de 85% et 95% préférentiellement de 88% à 94,5%, et encore plus préférentiellement de 90% à 94% en poids sec de sorbitol pulvérulent cristallisé.

De manière préférée, la composition édulcorante est caractérisée en ce qu'elle présente une enthalpie au plus égale à 146 J/g d'échantillon, et encore plus préférentiellement au plus égale à 142 J/g d'échantillon.

La composition édulcorante est aussi caractérisée en ce qu'elle présente plus préférentiellement un diamètre moyen volumique (moyenne arithmétique) D4,3, compris entre 250 et 350 μm, et de préférence entre 280 et 330 μm.

La composition peut être caractérisée en ce que le sorbitol pulvérulent cristallisé est constitué d'au moins 85% en poids, de préférence d'au moins 90% en poids, plus préférentiellement encore d'au moins 95% en poids de cristaux de forme y.

La composition édulcorante peut également être caractérisée en ce que sa valeur d'hygroscopicité, déterminée par son évolution pondérale entre 0% et 60% d'humidité relative est comprise entre 2,5 et 3,4%, de préférence entre 2,8% et 3,2%.

Cette composition édulcorante peut posséder une compressibilité inférieure à 25%, de préférence comprise entre 7 et 22% et plus préférentiellement encore entre 10 et 20%.

La présente invention a également pour objet l'utilisation de la dite composition édulcorante dans la fabrication de chewing-gums.

La présente invention concerne donc également une composition de chewing-gum contenant, les pourcentages étant indiqués en poids sec par rapport au poids sec total de la dite composition:

- de 10% à 28%, préférentiellement de 15% à 25%, et encore plus préférentiellement 20% d'au moins une gomme de base,

- de 20% à 70%, préférentiellement de 30% à 60 %, de la composition édulcorante décrite précédemment,

- de 0,1 % à 5 %, préférentiellement de 0,5 % à 3 %, et encore plus préférentiellement de 1 à 1,8 % d'au moins un arôme.

L'invention concerne l'utilisation de la composition édulcorante dans la fabrication de comprimés à usage pharmaceutique ou alimentaire.

## DESCRIPTION DETAILLEE DE l'INVENTION

La composition édulcorante pulvérulente conforme à l'invention est tout d'abord caractérisée en ce qu'elle comprend :

- de 80 à 95% en poids sec d'un sorbitol pulvérulent cristallisé
  et présente
- une enthalpie au plus égale à 150 J/g,
- un diamètre moyen volumique compris entre 200 et 350 $\mu$m, et
- une surface spécifique, déterminée selon la méthode BET, est inférieure à 0,6 m$^2$/g, de préférence comprise entre 0,15 et 0,4 m$^2$/g, et encore plus préférentiellement entre 0,20 et 0,35 m$^2$/g.

Dans la présente demande, l'expression « comprend entre 80 et 95% en poids sec d'un sorbitol pulvérulent cristallisé » signifie que la richesse en sorbitol pur ou teneur en sorbitol pur dans la composition édulcorante est de de 80 à 95% en poids sec. Le reste étant constitué par des sucres réducteurs totaux comme le mannitol, le maltitol ainsi que des DP3 voire des DP4.

De manière préférée, la composition édulcorante pulvérulente conforme à l'invention est caractérisée en ce qu'elle présente une teneur en sorbitol comprise entre 85% et 95%, préférentiellement comprise entre 88% et 94,5%, et encore plus préférentiellement comprise entre 90% et 94%.

Au sens de l'invention, le sorbitol contenu dans la composition édulcorante est sous une forme cristalline essentiellement $\gamma$. Au sens de l'invention, on entend par « forme cristalline essentiellement $\gamma$ », une teneur en cristaux de sorbitol de forme $\gamma$ supérieure à 85% en poids, de préférence supérieure à 90% en poids, plus préférentiellement encore supérieure à 95% en poids.

Dans un mode particulièrement préféré de l'invention, la teneur en cristaux de sorbitol de forme $\gamma$ dans la composition édulcorante est supérieure à 98% en poids.

La composition édulcorante selon l'invention présente une enthalpie, ou plus précisément une enthalpie de fusion ou chaleur latente de fusion, au plus égale à 150 J/g.

L'enthalpie est déterminée par calorimétrie différentielle à balayage (ou DSC Differential Scanning Calorimetry en anglais).

La Differential Scanning Calorimetry (DSC) fonctionne selon une rampe de température. Elle mesure l'énergie fournie pour respecter la montée en température de l'échantillon de sorbitol poudre. Tant que le produit est stable, sa température évolue linéairement en fonction de la rampe à laquelle il est soumis.

Lorsque le produit atteint sa température de changement de phase, il consomme une nouvelle énergie pour passer sous forme liquide. L'énergie supplémentaire nécessaire au respect de la rampe est enregistrée par l'appareil de mesure. Il s'agit d'une enthalpie.

On a tendance à considérer que l'enthalpie de fusion représente l'énergie qu'il faut fournir pour passer de l'état cristal à un état amorphe. Ainsi une forme cristalline majoritaire aura une enthalpie plus élevée, et donc il sera plus difficile de passer de ladite forme cristalline à la forme amorphe.

Au contraire, un mélange moins pur contenant à la fois une forme cristalline et une forme amorphe aura une enthalpie plus faible car il est plus facile de passer à l'état amorphe.

Ainsi, un sorbitol de très haute pureté, supérieur à 96% en poids sur sec aura donc une enthalpie de fusion élevée car il contient peu, voire quasiment pas d'impuretés.

De manière préférée, la composition édulcorante selon l'invention possède une enthalpie au plus égale à 146 J/g d'échantillon ou encore plus préférentielle, au plus égale à 142 J/g d'échantillon.

A titre d'exemple, le sorbitol commercialisé par la société Demanderesse sous la marque NEOSORB® présente une enthalpie de fusion de l'ordre de 165 J/g d'échantillon.

La composition édulcorante conforme à l'invention est également caractérisée par sa granulométrie particulière. Ainsi la composition selon l'invention présente un diamètre moyen volumique (moyenne arithmétique) D4,3 compris entre 200 et 350 $\mu$m.

Dans un mode préférentiel, le diamètre moyen volumique (moyenne arithmétique) D4, 3 est compris entre 250 et 350 $\mu$m ou plus préférentiellement entre 280 et 330 $\mu$m.

Le choix de la granulométrie des poudres de polyols, notamment des poudres de sorbitol est très important. Les particules de sorbitol possèdent une structure microscopique dendritique, c'est-à-dire comme un enchevêtrement d'aiguilles. En raison de cette structure particulière, il a généralement été constaté que l'utilisation de sorbitol poudre présentant une granulométrie moyenne supérieure à 200 micromètres dans la fabrication de comprimés, tablettes et/ou chewing-gums confère aux dits produits une texture dite sableuse, notamment aux chewing-gums (en particulier lors de la mastication).

La composition édulcorante conforme à l'invention, bien que présentant une granulométrie plus importante (une granulométrie moyenne supérieure à 200 $\mu$m) n'a pas cet effet négatif. En effet, comme démontré ci-après, les chewing-gums fabriqués à partir de cette composition ne présentent pas cette texture désagréable sableuse en bouche.

Ces valeurs de diamètre moyen ou diamètre volumique moyen sont déterminées par un granulomètre à diffraction LASER type LS 230 de la société BECKMAN-COULTER, équipé de son module de dispersion poudre (voie sèche), en suivant le manuel technique et les spécifications du constructeur. Les conditions opératoires de vitesse de vis sous trémie et d'intensité de vibration de la goulotte de dispersion sont déterminées de manière à ce que la concentration optique soit comprise entre 4% et 12%, idéalement 8%. La gamme de mesure du granulomètre à diffraction LASER type LS 230 est de 0,04 $\mu$m à 2.000 $\mu$m. Les résultats sont exprimés en $\mu$m.

La composition édulcorante conforme à l'invention est également caractérisée en ce qu'elle présente une surface spécifique plus faible qu'un sorbitol du commerce.

En effet, la composition édulcorante selon l'invention est caractérisée par une surface spécifique, déterminée selon la méthode BET, inférieure à 0,6 m$^2$/g, de préférence comprise entre 0,15 et 0,4 m$^2$/g et plus préférentiellement comprise entre 0,20 et 0,35 m$^2$/g.

On détermine la surface spécifique de la composition édulcorante selon l'invention grâce à un analyseur de surface spécifique de marque BECKMAN-COULTER, de type SA3100 basé sur un test d'absorption de l'azote sur la surface du produit soumis à l'analyse, en suivant la technique décrite dans l'article BET Surface Area by Nitrogen Absorption de S. BRUNAUER et al. (Journal of American Chemical Society, 60, 309, 1938). Le calcul de la surface spécifique est basé sur la théorie de l'adsorption multicouche. Pour plus de détails sur cette théorie, on pourra se référer au livre de P.W. Atkins [ATKINS et MORROW, 1986).

L'analyse BET est réalisée en 3 points.

Par définition, la surface spécifique (*Ss*) appelée aussi « Aire massique » représente la surface totale (As) par unité de masse (M) et on l'exprime généralement en m$^2$/g.

La surface spécifique désigne la superficie réelle de la surface d'un objet par opposition à sa surface apparente.

La surface spécifique dans la présente invention est mesurée sur une coupe de 250 $\mu$m à 841 $\mu$m.

L'agent de charge avant d'être soumis à une analyse de sa surface spécifique subit un tamisage sur des tamis dont la granulométrie est comprise entre 250 $\mu$m et 841 $\mu$m. Cela permet d'éliminer toutes les particules dont le diamètre est inférieur à 250 $\mu$m et également toutes les particules dont le diamètre est supérieur à 841 $\mu$m.

Cela permet par conséquent de se concentrer sur la coupe granulométrique concernant la distribution majoritaire des poudres d'agent de charge et de s'affranchir des fines et des particules trop grosses qui fausseraient l'analyse.

Dans le cas d'une poudre, la surface réelle est la somme des surfaces des grains. De manière générale, pour une masse ou un volume donné, plus les grains sont fins et plus la surface spécifique est importante.

De manière très surprenante, la composition édulcorante conforme à la présente invention présente une surface spécifique basse comprise entre 0,20 et 0,35 m$^2$/g, pour un diamètre moyen volumique (moyenne arithmétique) D4, 3 compris entre 200 et 350 $\mu$m.

On aurait dû s'attendre à l'inverse. Avec un tel diamètre moyen volumique, la surface spécifique aurait normalement dû être plus élevée, proche de 1 m$^2$/g.

A la connaissance de la société Demanderesse, il n'existe pas dans le commerce de composition édulcorante conforme à celle de l'invention avec des paramètres de surface spécifique et de granulométrie similaires. La composition édulcorante selon l'invention va à l'encontre des préjugés existant qui corrèlent la granulométrie d'une poudre ou diamètre des particules de la dite poudre avec sa surface spécifique.

La composition édulcorante conforme à l'invention peut également être caractérisée par son hygroscopicité ou une valeur d'hygroscopicité, déterminée par son évolution pondérale entre 0% et 60% d'humidité relative (HR), comprise entre 2,5 et 3,4%, de préférence comprise entre 2,8 et 3,2%.

Le test de mesure de l'hygroscopicité utilisé dans la présente invention est le même que celui décrit dans le brevet EP 1008602. Ainsi ce test consiste à évaluer la variation de poids de l'échantillon de composition édulcorante lorsqu'il est soumis à différentes taux d'humidité résiduelle (HR) entre 0 % et 60% à 20°C dans un équipement fabriqué par la société SURFACE MEASUREMENT SYSTEMS (Londres UK) et dénommé Dynamic Vapour Sorption Série 1.

Cet équipement consiste en une microbalance différentielle qui permet de quantifier l'évolution pondérale d'un échantillon par rapport à une référence (ici la nacelle de référence de la balance différentielle est vide) lorsque celui-ci est soumis à différents taux d'humidité.

Le gaz vecteur est l'azote, et le poids de l'échantillon est compris entre 10 et 12 mg. Les HR programmées sont 0 % HR pendant 24 h (déshydratation) puis 10, 20, 30, 35, 40, 45, 50, 52, 54, 56, 58 et 60 % HR. Le facteur de stabilité qui permet le passage automatique d'une HR à la suivante est le rapport dm/dt qui est fixé à 0,002 %/mn pendant 20 minutes.

En final, est obtenue une table de valeurs correspondant pour chaque HR à l'équation $[(m-m_0)/m_0]$ x 100 où « m » est la masse de l'échantillon en fin de test pour l'HR considérée et « $m_0$ » la masse en fin de déshydratation.

Les résultats sont exprimés comme la différence entre les valeurs d'évolution pondérale (telles que décrites ci-dessus) obtenues respectivement après déshydratation (à 0 % HR) puis à 60 % d'HR.

A nouveau, il est particulièrement surprenant que la composition édulcorante conforme à l'invention puisse présenter conjointement une surface spécifique inférieure à $0,6m^2/g$ et une hygroscopicité comprise entre 2,5 et 3,4%. En effet, il est admis très classiquement que l'hygroscopicité d'un produit pulvérulent augmente avec sa surface spécifique, i.e. sa surface exposée au milieu contenant de la vapeur d'eau.

Or, la composition édulcorante hydrogénée conforme à l'invention présente une surface spécifique faible, caractéristique d'un produit cristallisé, avec pourtant une hygroscopicité relativement élevée, caractéristique d'un produit granulé.

A titre d'exemples, le sorbitol commercialisé par la société MERCK sous l'appellation SORBITOL grade L présente une hygroscopicité de 2,4 % dans les mêmes conditions de mesure pour une surface spécifique selon BET de 1,55 $m^2/g$, et le sorbitol commercialisé par la société Demanderesse sous la marque NEOSORB® présente une hygroscopicité d'une valeur de 1,53 % pour une surface spécifique inférieure à $1m^2/g$.

De façon surprenante et inattendue et contrairement à ce que l'on attendait, la composition édulcorante conforme à l'invention présente une hygroscopicité remarquablement plus élevée que ce qui est classiquement décrit pour les sorbitols pulvérulents du commerce présentant également des surfaces spécifiques plus élevées.

En raison de l'hygroscopicité plus élevée de notre composition édulcorante par rapport à celle des sorbitols du commerce, on pouvait s'attendre à des problèmes de mottage. Or, contre toute attente, la composition édulcorante conforme à l'invention est caractérisée par son absence de mottage, c'est-à-dire par son absence à former des agrégats lors de son stockage par exemple.

Le mottage (ou *caking* en anglais) est une dénomination générale de la modification des propriétés d'écoulement des poudres, comprenant à la fois la formation de petits agrégats (facilement friables ou durs), le collage voire la prise en masse totale de la poudre. La formation de liens entre les particules, due à la solidification d'une solution sursaturée à la surface des particules, est le mécanisme responsable du mottage des poudres. Le mottage des composés cristallins à l'état pulvérulent est dépendant de la granulométrie des particules constituant la poudre car les cristaux de faible dimension ont une surface spécifique élevée, et donc, une adsorption élevée d'eau, ce qui entraine une dissolution suivie d'une cristallisation. La formation de ces petits cristaux sert de ciment à la prise en masse de la poudre.

Or la composition édulcorante conforme à l'invention ne motte pas et cela peut s'expliquer en partie par sa surface spécifique faible.

Cette composition est donc tout à fait apte à être conditionnée en big bags et/ou en vrac.

En effet, la composition édulcorante selon l'invention a passé avec succès le test de simulation au stockage mis en place par la Demanderesse.

Ce test permet de simuler un stockage de produit en big bags. Pour réaliser ce test, 1200g (=m0) de produit à tester sont introduits dans un sachet en polyéthylène de 100 $\mu$m d'épaisseur.

Les dimensions approximatives du sachet vide sont 324 mm sur 209 mm.

Lorsque le produit est dans le sachet, on le ferme hermétiquement en chassant le maximum d'air occlus.

Ce sachet est ensuite placé dans un cylindre métallique de 220 mm de hauteur et 130 mm de diamètre, perforé sur toute sa surface de trous de 8 mm de diamètre, disposés en quinconce avec une distance de 12 mm entre les centres des trous voisins.

Un disque métallique de diamètre juste inférieur à celui du cylindre est placé sur le sachet. Sur ce disque est placé un poids de 6,6 kg soit l'équivalent d'une pression d'environ 600kg/$m^2$, pression identique à celle que subit la poudre située au fond d'un big bag.

L'ensemble est ensuite placé dans une enceinte climatique réglée de manière à imposer 15 cycles de 8 heures (3,5 heures à une température de 15°C et 85% H.R. ; 0,5 heure de transition ; 3,5 heures à une température de 30°C et 85% H.R. ; 0,5 heure de transition).

A la fin de ces 15 cycles, le poids de 6,6 kg et le disque sont retirés, puis le sachet est sorti délicatement du cylindre et ouvert.

La totalité de la poudre est ensuite introduite délicatement dans un tonnelet de 5 litres qui est mis en rotation, à la vitesse maximum, pendant une minute, par un mélangeur à retournement MIXOMAT A14 (Fuchs/Suisse).

Le tonnelet est ensuite ouvert et vidé de sa poudre sur un tamis dont les mailles ont des ouvertures carrées d'environ 8 mm sur 8 mm.

Quelques légères secousses sur le tamis permettent d'éliminer toute la poudre dont les grains ont une taille inférieure à la taille des mailles du tamis.

On ne récupère ainsi que les mottes de produit retenues sur ce tamis, ces mottes sont pesées. On détermine alors la masse m1.

Le ratio (poids des mottes récupérées/poids de produit initial)*100 exprime le taux de produit motté.

Selon le test de mottage décrit ci-dessus, la composition édulcorante conforme à l'invention présente un ratio de produit motté d'environ 5%. Cela signifie donc que dans des conditions de simulation au stockage en conditionnement de type big bags, seulement environ 5% du produit a formé des agrégats, ce qui est extrêmement faible.

Il est particulièrement surprenant que la composition édulcorante selon la présente invention, bien que présentant une valeur d'hygroscopicité, comprise entre 2,5 et 3,4% et une richesse ou teneur en sorbitol n'excédant pas les 95% ne présente pas d'aptitude au mottage plus importante qu'un sorbitol du commerce, plus pur et possédant une plus faible hygroscopicité. En effet, traditionnellement les compositions de sorbitol trouvées dans le commerce ont des ratios de produit motté similaires à ceux trouvés pour notre composition, mais pour des valeurs d'hygroscopicité beaucoup plus faibles et des richesses en sorbitol bien plus élevées, de l'ordre de 98% en poids sur sec.

Il est en effet bien connu que plus un produit est pur, plus il est sous une forme cristallisé et donc moins il aura tendance à motter.

La composition édulcorante conforme à l'invention peut également être caractérisée par sa très bonne comprimabilité.

La comprimabilité se définit comme la mesure de l'aptitude d'une poudre à donner un comprimé lorsqu'on exerce une pression. Cette comprimabilité se mesure par le test de comprimabilité qui consiste à exprimer la dureté de comprimés en fonction de la force de compression exercée pour les obtenir.

En général, on dira qu'un produit est plus comprimable qu'un autre si ce produit permet d'obtenir des comprimés de dureté plus élevée à force de compression identique.

Le test de comprimabilité est réalisé sur une presse rotative de marque FETTE type PI000 équipée de poinçons ronds (diamètre 10 mm), concaves (rayon de courbure de 9 mm).

Avant l'étape de compression, le produit évalué est mélangé avec 0,5 % de stéarate de magnésium qui joue le rôle de lubrifiant.

Pour obtenir les différentes forces de compression, l'épaisseur du comprimé est maintenue constante (5 mm), par contre le poids est variable (à chaque force de compression correspond un poids de comprimé).

Pour évaluer la dureté du comprimé, les comprimés formés sont ensuite placés dans un duromètre de marque ERWEKA type 425. Cet appareil donne la valeur de la force en Newtons nécessaire pour rompre/écraser le comprimé.

Les profils de compression obtenus pour la composition édulcorante selon la présente invention en comparaison à un sorbitol de type NEOSORB® P60W par exemple, commercialisé par la Demanderesse sont quasiment superposables. Cela signifie que ces deux poudres ont une comprimabilité comparables, et que la composition édulcorante de l'invention est tout aussi comprimable qu'une poudre de sorbitol de l'art antérieur possédant une teneur en sorbitol bien supérieure, et dont le coût de fabrication est également plus élevé. La composition édulcorante conforme à l'invention peut également être caractérisée par sa vitesse de dissolution qui est inférieure à 15 secondes. En effet, la composition édulcorante possède une vitesse de dissolution élevée, traduisant également son excellente mouillabilité.

Pour mesurer cette vitesse de dissolution, on utilise un bécher de contenance 250 ml (forme basse) dans lequel on introduit 150 g d'eau déminéralisée dégazée à 20°C +/- 2°C. On pèse exactement 5 g de poudre de composition édulcorante hydrogénée. A t=0h on introduit rapidement et en une seule fois les 5 g d'échantillon et on déclenche le chronomètre. On mesure le temps nécessaire pour que l'échantillon se dissolve complètement, c'est-à-dire pour qu'il ne reste plus de particule en suspension. Le test est réalisé sous agitation douce à 200 rpm en utilisant un agitateur magnétique.

Ainsi dans les conditions du test de dissolution décrit ci-dessus, la composition édulcorante conforme à l'invention possède une vitesse de dissolution comprise entre 3 et 15 secondes, de préférence comprise entre 5 et 14 secondes et plus préférentiellement comprise entre 7 et 13 secondes.

La composition édulcorante peut être également caractérisée par une densité aérée supérieure à 0,600, de préférence comprise entre 0,610 et 0,700, plus préférentiellement comprise entre 0,630 et 0,660, et une densité tassée comprise entre 0,660 et 0,850, de préférence entre 0,700 et 0,800.

La composition édulcorante conforme à l'invention présente ainsi une compressibilité inférieure à 25%, de préférence comprise entre 7 et 22%, plus préférentiellement encore entre 10 et 20 %.

Une telle valeur de compressibilité confère à la composition édulcorante une meilleure stabilité de son état pulvérulent au stockage. Les valeurs de compressibilité obtenues pour la composition édulcorante selon l'invention traduisent les propriétés d'un produit qui s'écoule correctement.

Les valeurs de densité tassée et aérée, et de compressibilité, de la composition édulcorante hydrogénée selon l'invention sont déterminées en utilisant l'appareil POWDER TESTER type PTE commercialisé par la société HOSOKAWA, en suivant les spécifications du constructeur (réglage par défaut sur 180 secousses pour la mesure de la densité tassée).

Cet appareil permet de mesurer, dans des conditions standardisées et reproductibles, l'aptitude à l'écoulement d'une poudre en mesurant notamment la densité aérée vrac et la densité tassée vrac et ensuite de calculer, à partir de ces données, les valeurs de compressibilité par la formule suivante :

$$\text{Compressibilité}(\%) = \frac{(\text{densité tassée} - \text{densité aérée})}{\text{Densité tassée}} \times 100$$

Ainsi les compositions édulcorantes selon la présente invention ont une résistance au mottage particulièrement importante mais aussi de très bonnes caractéristiques d'écoulement (compressibilité), et de densité vis-à-vis des poudres de sorbitol de l'état de la technique.

Grâce à ces très nombreuses propriétés jusqu'alors jamais réunies pour une poudre de sorbitol, la composition édulcorante objet de la présente invention peut avantageusement être employée dans un procédé de fabrication de chewing-gum, ainsi qu'en compression ou pour la fabrication de comprimés.

Dans la présente invention, le terme « chewing-gum » est utilisé indifféremment pour désigner les chewing-gums et les bubble-gums. La différence entre ces deux types étant d'ailleurs assez floue. On a coutume de dire que les chewing-gums se mâchent alors que les bubble-gums sont destinés à faire des bulles, et donc sont traditionnellement plutôt consommés par un jeune public.

Ladite composition est particulièrement adaptée pour une utilisation dans les procédés de fabrication de chewing-gums, notamment du fait de son bon écoulement et de sa faible tendance à créer des poussières mais pas seulement.

La présente invention concerne donc également l'utilisation de la composition édulcorante dans la fabrication de chewing-gums.

La Société Demanderesse a démontré que la composition édulcorante selon l'invention avait un intérêt particulièrement en termes de réduction des coûts de formulation dans une recette de chewing-gum.

Ladite composition permet notamment une réduction de 60%, de préférence de 50%, et encore de préférence de 40% de la gomme de base comparativement à une composition de chewing-gum de l'art antérieur ou classique, sans impacter sur les qualités finales organoleptiques du produit fini.

Qui plus est, ladite composition permet donc une réduction non négligeable de la teneur en arômes utilisés. En effet, le fait de mettre moins de gomme de base dans la recette impacte directement sur la quantité d'arômes à ajouter.

L'utilisation de ladite composition permet donc en outre une réduction de 50%, de préférence de 40%, et encore de préférence de 25% de la quantité d'arômes comparativement à une composition de chewing-gum de l'art antérieur ou classique.

En effet, la Société Demanderesse a notamment réussi à démontrer que l'utilisation de la composition édulcorante de la présente invention, dans une formulation de type chewing-gum permet de conférer au chewing-gum une texture finale plus souple que celle des chewing-gums obtenus selon la même recette mais en mettant en oeuvre un sorbitol de l'art antérieur. Etant donné que c'est la gomme de base qui permet en grande partie de conférer la texture au chewing-gum, la société Demanderesse a alors eu l'idée de réduire la quantité de gomme de base de façon à ne pas modifier la texture finale du chewing-gum. L'utilisation de la composition édulcorante de la présente invention permet en outre de réduire la quantité d'arômes traditionnellement utilisée. En effet une partie des arômes reste emprisonnée dans la gomme de base lors de la mastication et ces arômes ne sont donc jamais relargués dans la salive.

L'intérêt de l'utilisation de la composition édulcorante est donc double puisqu'elle permet d'une part la réduction du taux de gomme de base, ce qui permet par conséquent de diminuer d'autre part la quantité d'arômes utilisés. De telles diminutions de quantité de gomme et d'arômes engendrent une réduction importante au niveau des coûts de fabrication, et sont donc très intéressantes pour les industriels.

Les propriétés particulières de la composition édulcorante selon l'invention, et plus précisément sa surface spécifique faible, inférieure à 0,6 m$^2$/g selon la méthode BET, combinée à une teneur en sorbitol n'excédant pas les 95%, et à un diamètre moyen volumique compris entre 200 et 350 $\mu$m confèrent à cette composition édulcorante hydrogénée la faculté d'assouplir la gomme de base et donc le chewing-gum au final.

Par ailleurs, bien que possédant une quantité moins importante d'arômes dans la recette, la perception des arômes, tant en intensité qu'en persistance, dans le chewing-gum renfermant la composition édulcorante selon l'invention au moins identique au chewing-gum selon l'art antérieur.

La société Demanderesse a notamment démontré qu'en réduisant la gomme de base, il était tout à fait possible d'obtenir des chewing-gums tout à fait satisfaisants en terme de texture. Ceci n'était pas du tout évident car les proportions entre les différents constituants sont généralement figées et qu'il n'est pas possible de les modifier sans impacter de façon négative la qualité finale des produits.

La présente invention consiste donc également en une composition de chewing-gum contenant, les pourcentages étant indiqués en poids sec par rapport au poids sec total de la dite composition:

- de 10% à 28%, préférentiellement de 15% à 25%, et encore plus préférentiellement 20% d'au moins une gomme de base,

- de 20% à 70%, préférentiellement de 30% à 60 %, de la composition édulcorante décrite précédemment,

- de 0,1 % à 5 %, préférentiellement de 0,5 % à 3 %, et encore plus préférentiellement de 1 à 1,8 % d'au moins un arôme.

La Demanderesse recommande d'effectuer ce mélange à une température comprise entre 45°C et 80°C, préférentiellement dans un pétrin à bras en Z avec une double enveloppe ou dans un mélangeur en continu. De manière préférée, il convient de chauffer au préalable la gomme de base, à une température comprise entre 45°C et 80°C, de préférence entre 45°C et 55°C, par tout moyen connu de l'homme du métier. On pourra à titre d'exemple la chauffer dans un four à micro-ondes ou dans une étuve.

Le mélange entre les composés précités peut en outre mettre en oeuvre un autre polyol en tant qu'agent sucrant, sous forme de poudre ou de liquide, tel que par exemple le mannitol, le maltitol, le xylitol, l'erythritol, le lactitol, l'isomalt, les sirops de maltitol, les sirops de sorbitol, les sirops de glucose hydrogénés.

Dans un mode de réalisation avantageux de l'invention, la composition édulcorante hydrogénée peut également être associée à un sirop de sorbitol dans la recette de fabrication de chewing-gums.

De manière intéressante, la combinaison de la composition édulcorante hydrogénée de l'invention à un sirop de sorbitol de pureté inférieure à 96% permet également de diminuer la quantité de gomme de base dans les chewing-gums, et donc d'arômes.

Le mélange entre les composés précités peut aussi mettre en oeuvre, à raison d'au plus 5 % en poids par rapport au poids total du chewing-gum, au moins un constituant choisi parmi les colorants, les édulcorants intenses tels qu'aspartame, acésulfame K, alitame, néotame, sucralose, saccharine, néohespéridine DC, stiéviosides, brazzéine..., les actifs pharmaceutiques, minéraux, extraits de plantes, anti-oxydants, et fibres indigestibles telles que par exemple les oligosaccharides tels que les fructo oligosaccharides, les fibres indigestibles telles que le Fibersol™ commercialisé par la société MATSUTANI, ou encore le NUTRIOSE® commercialisé par la Demanderesse, des émulsifiants comme la lécithine, etc...

La gomme de base utilisée pourra être adaptée au type de chewing-gum fabriqué. Elle pourra comprendre des élastomères synthétiques et/ou naturels comme le polyisoprène, l'acétate de polyvinyle, le polyisobutylène, des latex, des résines comme les résines terpéniques, les esters et les alcools de polyvinyle, des matières grasses ou des cires comme par exemple la lanoline, les huiles végétales partiellement hydrogénées ou non, les acides gras, les esters partiels de glycérol, la paraffine, les cires microcristallines.

Dans la fabrication de la composition de chewing-gum, l'étape de mélange des ingrédients précités est suivie des étapes d'extrusion, laminage, découpe, refroidissement puis conditionnement, réalisées selon toutes les techniques bien connues de l'homme du métier. Au final, le chewing-gum est présent sous une des formes bien connues de l'homme du métier, telles que tablettes, billes, dragées, cubes ou encore de comprimés.

La présente invention concerne également l'utilisation de la composition édulcorante dans la fabrication de comprimés à usage pharmaceutique ou alimentaire.

L'invention concerne également outre des compositions de chewing-gum ou chewing-gum, un comprimé fabriqué à partir de la composition édulcorante conforme à l'invention. La teneur en composition édulcorante du comprimé dépendra de l'usage souhaité dudit comprimé. Typiquement la teneur en composition édulcorante du comprimé pourra être comprise entre 1% et 90% en poids sec.

La composition édulcorante conforme à l'invention est susceptible d'être obtenue par :

- hydrogénation d'un sirop de glucose présentant entre 40 et 50 % de matière sèche et comprenant entre 80 et 95% de glucose, entre 3,5 et 12 % de DP2 et entre 0,5 et 8 % de DP3 ;
- suivi d'une étape de concentration du dit sirop hydrogéné jusqu'à une matière sèche supérieure à 50 % et de préférence entre 70 et 80 % ;
- d'une étape de cristallisation, plus particulièrement de granulation
- éventuellement suivie d'une étape de maturation, d'un broyage et enfin d'un tamisage.

Pour l'étape de granulation on procédera de manière tout particulièrement préférée à une pulvérisation du sirop hydrogéné ou riche en sorbitol sur une poudre de sorbitol (amorce) au sein d'un appareillage de type drageuse. On peut faire référence ici pour cette étape de granulation au document FR 2202867.

L'invention sera mieux comprise à la lecture des exemples suivants, qui ne sauraient limiter en rien la présente invention.

### Exemple 1

Procédé de fabrication de la composition édulcorante selon l'invention :

Un sirop de glucose à 45% de matière sèche et comprenant 95 % en poids sec de glucose, 4% de DP2 et 1% de DP3 est hydrogéné sous une pression d'hydrogène de 60 bars. Lors de l'hydrogénation, le pH décroit lentement à une valeur basse d'environ 4,5. Le pH est ensuite relevé à 8, par ajout de soude, sous la forme d'une solution aqueuse de soude, pour terminer l'hydrogénation jusqu'à une teneur en sucres réducteurs inférieure ou égale à 2% sur sec.
L'étape d'hydrogénation est suivie par :

- une étape de concentration à une teneur en matière sèche supérieure à 50 % de préférence entre 70 et 80 % de matière sèche ;

- une étape de cristallisation dans les conditions décrites dans le brevet FR 2202867,

- et enfin d'un broyage

### Exemple 2 :

Caractéristiques de la composition édulcorante pulvérulente selon l'invention

La composition obtenue selon la mise en oeuvre du procédé de fabrication décrit dans l'exemple 1 est analysée de façon à déterminer toutes ses caractéristiques physico-chimiques, et est comparée à un sorbitol poudre du commerce commercialisé par la Société Demanderesse sous la marque Neosorb®.

|  | Composition édulcorante selon l'invention | Sorbitol Neosorb® P60W |
|---|---|---|
| Sorbitol (%/sec) | 93,2 | 98,5 |
| Maltitol (%/sec) | 1,8 | 0,2 |
| Mannitol (%/sec) | 0,8 | 0,6 |
| Enthalpie (J/g) | 141 | 163 |
| Diamètre moyen volumique ($\mu$m) | 320 | 290 |
| Surface spécifique (m$^2$/g) | 0,3 | 0,85 |
| Hygroscopicité (%) | 2,9 | 1,6 |
| Densité aérée | 0,650 | 0,680 |
| Densité tassée | 0,710 | 0,710 |

C'est la première fois qu'une telle composition édulcorante présentant les caractéristiques réunies ci-dessus est décrite. Cette composition est donc nouvelle.

### Exemple 3

Mise en oeuvre de la composition édulcorante selon l'invention dans la fabrication de chewing-gums.
Le témoin a été réalisé avec un sorbitol poudre commercialisé par la Société Demanderesse sous la marque Neosorb®.
Tous les pourcentages exprimés le sont par rapport au poids sec total de la composition de chewing-gum mise en oeuvre.

#### 1. Préparation des compositions de chewing-gums

Ingrédients mis en oeuvre dans les compositions de chewing-gum :

| Ingrédients | Composition témoin (%) | Composition selon l'invention (%) |
|---|---|---|
| Gomme de base Solsona T | 30 | 20 |
| Mannitol 60 | 5 | 5 |
| Xylitol 90 | 7 | 7 |
| Sirop de maltitol Lycasin® 85/55 | 4 | 8 |
| Glycérine | 4 | 4 |
| Arôme liquide | 1,50 | 1,10 |
| Arôme poudre | 0,50 | 0,50 |
| Sorbitol NEOSORB® P60W | 48 | 0 |
| Composition édulcorante selon l'invention | 0 | 54,4 |
| TOTAL | 100 | 100 |

La gomme de base SOLSONA T est commercialisée par la société CAFOSA

Le Sorbitol NEOSORB® P60W est une poudre de sorbitol cristalline, commercialisée par la Demanderesse. Le Mannitol 60, Xylitol 90 et le sirop de maltitol Lycasin® 85/55 sont également commercialisés par la Demanderesse.

Mode opératoire pour la préparation des compositions de chewing-gum témoin et selon l'invention

- Mélange : procédure en minutes - Réalisé dans un pétrin bras en Z à 45°C - Fabrication de batch de 50 kg de centre

    0 min : Introduire la gomme de base fondue (étuvée une nuit à 50°C) et le mannitol et xylitol.

    3 min : Ajouter le Lycasin® 85/55.

    5 min : Ajouter soit le sorbitol NEOSORB®, soit la composition édulcorante hydrogénée selon l'invention.

    9 min : Ajouter la glycérine.

    10 min : Ajouter l'arôme en poudre.

    12 min : Ajouter l'arôme de menthe/vanille liquide.

    15 min : Décharger le pétrin (la pâte est à environ 50°C). Former des pains d'environ 2 kg et les stocker 1 heure à 50% d'HR et à 20°C. Les pains doivent être à environ 48°C pour l'extrusion.

- Extrusion (Appareil Togum TO - E82)

- Consigne de température du corps = 36°C

- Consigne de température de la tête = 39°C.

- Laminage 4 postes - Prédécoupe 2 postes (Appareil Togum TO - W191)

- Saupoudrage de la bande de chewing-gum avec un mélange 90/10 mannitol/talc.

- Maturation

- Stocker les plaques prédécoupées de coussinets à environ 15°C-50% HR pendant environ 24h.

## 2. Evaluation des qualités organoleptiques des chewing-gums

Les chewing-gums obtenus précédemment ont été dégustés par un panel de 15 personnes entraînées à la dégustation et à la notation des chewing-gums.

Le panel a été invité à noter de 0 à 4 la souplesse des chewing-gums lors des premières secondes de mâche mais également après trois minutes de mastication.

4 étant le maximum de souplesse et 0 correspondant à un chewing-gum très dur voire cassant. Le panel a également été invité à noter la perception de l'arôme pendant la mastication. Ce test a également été fait lors des premières secondes de mâche et après trois minutes de mastication afin d'évaluer la persistance de l'arôme.

4 étant la note donnée pour un arôme très fort et 0 correspondant à un chewing-gum ne possédant plus du tout d'arôme.

Les produits étaient présentés dans un ordre aléatoire, et codés avec un numéro à 3 chiffres afin que les panélistes ne soient pas influencés ni par la connaissance des produits ni par leurs codes. Les dégustations ont été faites dans un laboratoire d'analyse sensorielle.

A T+0, le chewing-gum est introduit dans la cavité buccale et en même temps le chronomètre est déclenché. Puis la mastication débute.

Le traitement des données a été effectué par un traitement statistique (ANOVA et tests de comparaison de moyennes sont effectués sur les moyennes obtenues à chaque intervalle de temps).

| | Evaluation de la souplesse | | Evaluation de la persistance de l'arôme | |
|---|---|---|---|---|
| | T=10 secondes | T=3 min | T=10 secondes | T=3 min |
| **CG témoin** | 4 | 3 | 4 | 2 |
| **CG selon l'invention** | 4 | 3 | 4 | 3 |

Il ressort que :

- la souplesse des deux chewing-gums évolue de manière identique. Bien que possédant 10% de gomme de base de moins que le chewing-gum témoin, aucune différence en terme de texture et plus particulièrement en terme de souplesse entre les deux échantillons n'a été perçue par le panel de dégustateurs.

- Au niveau de la perception et de la persistance de l'arôme, là encore il n'y a pas de différence à t=10 secondes entre le chewing-gum témoin et celui selon l'invention. Bien que possédant une quantité moins importante d'arômes dans la recette, la perception de ce dernier dans le chewing-gum renfermant la composition édulcorante selon l'invention est identique au chewing-gum témoin. Il semblerait même que la persistance de l'arôme soit améliorée dans le temps puisque le chewing-gum obtenue à partir de la composition édulcorante selon l'invention, contenant moins de gomme de base et moins d'arôme est pourtant noté comme étant légèrement meilleur que le chewing-gum témoin au bout de trois minutes de mastication.

- Ainsi le chewing-gum fabriqué avec la composition édulcorante selon l'invention et contenant 10% de gomme de base de moins sur poids, soit une réduction de cette dernière de 33%, et contenant 0,4% d'arôme de moins sur poids, soit une réduction de 20% de la quantité d'arôme est identique en termes de texture et est légèrement supérieur en termes de persistance d'arômes.

L'intérêt de la présente invention est parfaitement démontré par cet exemple.

## Exemple 4

4 autres essais ont été réalisés afin de tester l'intérêt de la composition édulcorante selon l'invention dans la réduction du taux de gomme de base dans une recette de chewing-gum et afin de comparer ladite composition selon l'invention avec d'autres polyols. Les 4 nouveaux essais ont été réalisés avec un taux de gomme de base réduit à 20% (en poids).

L'essai 1 concerne une recette de chewing-gums réalisés avec le Neosorb® P60W.

L'essai 2 concerne une recette de chewing-gums mettant en oeuvre la composition édulcorante selon l'invention.

L'essai 3 concerne une recette de chewing-gum réalisés avec du maltitol commercialisé par la Société Demanderesse sous la marque SweetPearl®, et possédant une granulométrie moyenne de 150 $\mu$m.

L'essai 4 concerne une recette de chewing-gums réalisés avec du maltitol commercialisé par la Société Demanderesse sous la marque SweetPearl® et possédant une granulométrie de 90 $\mu$m.

Tous les pourcentages exprimés le sont par rapport au poids sec total de la composition de chewing-gum mise en oeuvre.

*Préparation des compositions de chewing-gums*

Ingrédients mis en oeuvre dans les compositions de chewing-gum :

| INGREDIENTS | Composition Témoin (Exemple 3) | ESSAI 1 | ESSAI 2 | ESSAI 3 | ESSAI 4 |
|---|---|---|---|---|---|
| Sorbitol NEOSORB® P60W | 48,00 | 54,38 | | | |
| Composition édulcorante selon l'invention | | | 54,38 | | |
| SweetPearl® 150 | | | | 54,38 | |
| SweetPearl® 90 | | | | | 54,38 |
| Gum base Solsona T | 30,00 | 20,00 | 20,00 | 20,00 | 20,00 |
| Mannitol 60 | 5,00 | 5,00 | 5,00 | 5,00 | 5,00 |
| Xylitol 90 | 7,00 | 7,00 | 7,00 | 7,00 | 7,00 |
| Glycérine | 4,00 | 4,00 | 4,00 | 4,00 | 4,00 |
| Sirop de maltitol Lycasin® 85/55 | 4,00 | 8,00 | 8,00 | 8,00 | 8,00 |
| Arôme liquide | 1,50 | 1,13 | 1,13 | 1,13 | 1,13 |
| Arôme poudre | 0,50 | 0,50 | 0,50 | 0,50 | 0,50 |
| TOTAL | 100 | 100 | 100 | 100 | 100 |

Le mode opératoire pour la préparation des compositions de chewing-gum témoin et selon l'invention est exactement le même que celui décrit à l'exemple 3 ci-dessus.

**Evaluation du comportement des chewing-gums lors de leur préparation en batch**

Le témoin (composition témoin de l'exemple 3) se comporte très bien lors de la préparation des chewing-gums. La gomme est bien élastique et pas trop dure. Aucun phénomène de collage n'est à déplorer.

L'essai 1 (Neosorb® P60W et 20% gomme de base) présente une gomme de base extrêmement dure qui durcit de plus en plus dans le temps. La gomme est très compacte avec aucune élasticité. De plus, cet essai est très sec.

L'essai 2 (composition selon l'invention et 20% de gomme de base) présente un comportement identique à celui du témoin, à savoir : une gomme de base bien élastique et pas trop dure. Il n'y a aucun phénomène de collage à déplorer.

L'essai 3 (SweetPearl® 150 et 20% de gomme de base) possède une gomme de base extrêmement molle. Elle possède une bonne élasticité mais est très collante et difficile à mettre en forme.

L'essai 4 est identique à l'essai 3.

*Evaluation des qualités organoleptiques des chewing-gums*

Les chewing-gums obtenus précédemment ont été dégustés par un panel de 15 personnes entraînées à la dégustation et à la notation des chewing-gums.

Le panel a été invité à noter de 0 à 4 la souplesse des chewing-gums au bout de trois minutes de mastication.

4 étant le maximum de souplesse et 0 correspondant à un chewing-gum étant devenu très dur voire cassant.

La dureté a également été notée comme étant la résistance du chewing-gum lors de l'attaque de la mâche.

4 étant la note pour un chewing-gum présentant une résistance à la mâche et 0 étant pour un chewing-gum extrêmement mou dès l'attaque.

Le panel a également été invité à noter la perception de l'arôme pendant la mastication. Il a été demandé de chronométrer le moment où l'arôme était le plus intense. C'est ce qu'on appelle le pic aromatique. Ce pic est noté en seconde. Un pic aromatique précoce signifie une libération de l'arôme plus tôt lors de la mastication, et donc un appauvrissement en notes aromatiques également plus précoce.

L'intensité du pic aromatique a également été notée sur une échelle de 1 à 3.

3 étant la note donnée pour un pic aromatique important et 0 correspondant à un pic aromatique de faible intensité.

Et le dernier critère évalué est celui du volume occupé par le chewing-gum en fin de mâche. Plus le chewing-gum est gros plus la note est importante. 4 étant le maximum.

Les produits étaient présentés dans un ordre aléatoire, et codés avec un numéro à 3 chiffres afin que les panélistes ne soient pas influencés ni par la connaissance des produits ni par leurs codes. Les dégustations ont été faites dans un laboratoire d'analyse sensorielle.

A T+0, le chewing-gum est introduit dans la cavité buccale et en même temps le chronomètre est déclenché. Puis la mastication débute.

Le traitement des données a été effectué par un traitement statistique (ANOVA et tests de comparaison de moyennes sont effectués sur les moyennes obtenues à chaque intervalle de temps).

Il ressort que :

|  | Dureté | Volume | Souplesse | Tps pour le pic (s) | Intensité du pic |
|---|---|---|---|---|---|
| Témoin | 3,68 | 3,16 | 2,95 | 31,94 | 2,04 |
| Essai 1 | 3,48 | 2,44 | 2,77 | 22,59 | 2,03 |
| Essai 2 (invention) | 3,49 | 2,58 | 2,93 | 30,07 | 2,40 |
| Essai 3 | 0,98 | 1,78 | 2,98 | 24,05 | 2,72 |
| Essai 4 | 0,70 | 1,78 | 3,73 | 24,59 | 2,72 |

Le chewing-gum témoin est relativement dur, présente un volume important en fin de mâche et a un pic aromatique plus tardif. Cela signifie que la persistance de l'arôme sera également plus longue lors de la mâche.

Les deux essais réalisés avec du maltitol de deux granulométries différentes donnent des chewing-gums qui ne sont pas du tout satisfaisants. Ils ont été jugés beaucoup trop mous et ne présentant aucune dureté et résistance lors de l'attaque par la mâche. De plus le volume en fin de mâche est très petit, ce qui signifie qu'il n'y a eu aucune expansion lors de la mâche. L'apparition du pic aromatique est plus précoce. L'intensité aromatique perçue est la plus importante. Forcément dans ces deux essais, le chewing-gum étant beaucoup trop mou, on peut s'imaginer facilement que l'accès aux molécules aromatiques présentes dans la gomme de base est plus facile.

L'essai 1 est satisfaisant au niveau des caractéristiques de dureté et de volume en fin de mâche. Le pic aromatique apparait relativement tôt et donc la persistance de l'arôme est moindre au cours du temps. Qui plus est, l'intensité du pic aromatique est relativement faible.

L'essai 2 contenant la composition édulcorante selon l'invention est l'essai qui donne le plus de satisfaction. Bien que ne contenant que 20% de gomme de base, il possède un pic aromatique tardif traduisant une bonne perception de l'arôme lors de la mâche. De plus, l'intensité du pic est également élevée et témoigne d'une excellente perception des arômes. Tout cela pour un chewing-gum qui présente une bonne dureté lors de l'attaque. Enfin, le volume obtenu à la fin de la mâche témoigne également que le chewing-gum s'est bien expansé lors de sa mastication.

Ainsi le chewing-gum fabriqué avec la composition édulcorante selon l'invention et contenant 10% de gomme de base de moins sur poids, soit une réduction de cette dernière de 33%, et contenant 0,37% d'arôme de moins sur poids, soit une réduction de 18,5% de la quantité d'arôme est identique en termes de texture et est légèrement supérieur en termes de perception aromatique qu'un chewing-gum fabriqué selon l'art antérieur.

L'intérêt de la présente invention est parfaitement démontré par cet exemple.

## Exemple 5

Mise en oeuvre de la composition édulcorante selon l'invention dans la fabrication de chewing-gums en association avec un sirop de sorbitol.

### *Préparation des compositions de chewing-gums*

Ingrédients mis en oeuvre dans les compositions de chewing-gum :

| Recette | Témoin | Essai 1 | Essai 2 |
|---|---|---|---|
| Sorbitol NEOSORB® P60W | 38,40 | 44,40 | 0,00 |
| Composition édulcorante selon l'invention | 0,00 | 0,00 | 44,40 |
| Sorbitol liquide à 95% de pureté | 89,60 | 103,60 | 103,60 |
| Gomme de base Solsona T | 60,00 | 40,00 | 40,00 |
| Glycérine | 8,00 | 8,00 | 8,00 |
| Arôme liquide | 3,00 | 3,00 | 3,00 |
| Arôme poudre | 1,00 | 1,00 | 1,00 |
| TOTAL | 200 | 200 | 200 |

Tous les pourcentages exprimés le sont par rapport au poids sec total de la composition de chewing-gum mise en oeuvre.

Les chewing-gums ont été préparés selon le même procédé que celui décrit dans les exemples précédents.

Les chewing-gums ont également été dégustés par un jury entrainé selon les critères indiqués dans l'exemple 3 ci-dessus.

Il ressort que :

La souplesse des trois chewing-gums évolue de manière identique. Bien que possédant 10% de gomme de base de moins que le chewing-gum témoin, aucune différence en termes de texture et plus particulièrement en termes de souplesse entre les trois échantillons n'a été perçue par le panel de dégustateurs.

Au niveau de la perception aromatique, le chewing-gum de l'essai 1 a été noté comme étant celui qui possédait la note aromatique la plus importante et une persistance dans la durée également plus longue.

Ainsi le chewing-gum fabriqué avec la composition édulcorante selon l'invention et contenant 10% de gomme de base de moins sur poids, soit une réduction de cette dernière de 33%, est identique en termes de texture et est légèrement supérieur en termes de persistance d'arômes. L'intérêt de la présente invention est une fois de plus parfaitement démontré par cet exemple.

## Exemple 6

### Mesure des surfaces spécifiques selon l'invention

Comme indiqué ci-dessus, la surface spécifique selon l'invention, est mesurée sur un échantillon de poudre obtenu sur une coupe de 250 µm à 841 µm.

### Préparation de l'échantillon

Pour préparer l'échantillon, une quantité suffisante d'échantillon est tamisée sur les tamis de 841 µm et 250 µm afin de récupérer environ 3 grammes d'une coupe granulométrique comprise entre 841 et 250 microns.

Dans une cellule de mesure de l'appareil, préalablement séchée et tarée à 0,001 g près, on introduit ensuite une prise d'essai suffisante pour remplir le réservoir de la cellule au ¾.

### Dégazage

On place la cellule contenant l'échantillon au poste de dégazage.

On effectue le dégazage en se reportant au manuel d'utilisation de l'appareil.

### Analyse de la poudre

Le dégazage effectué, on pèse à nouveau la cellule à 0,001 g près et on la place au poste de mesure. L'analyse est ensuite effectuée en se reportant au manuel d'utilisation de l'appareil.

L'appareil traite automatiquement les résultats recueillis. Le résultat est exprimé en $m^2/g$.

**Revendications**

1.  Composition édulcorante **caractérisée en ce qu'**elle présente :

    - de 80 à 95% en poids sec de sorbitol pulvérulent cristallisé,
    - une enthalpie au plus égale à 150 J/g,
    - un diamètre moyen volumique compris entre 200 et 350 $\mu$m,
    - une surface spécifique, déterminée selon la méthode BET, est inférieure à 0,6 m$^2$/g, de préférence comprise entre 0,15 et 0,4 m$^2$/g, et encore plus préférentiellement entre 0,20 et 0,35 m$^2$/g.

2.  Composition édulcorante selon la revendication 1, **caractérisée en ce qu'**elle présente entre 88% et 94,5%, et plus préférentiellement entre 90% et 94 % de sorbitol pulvérulent cristallisé.

3.  Composition édulcorante selon la revendication 1 ou 2, **caractérisée en ce que** l'enthalpie est au plus égale à 146 J/g d'échantillon, et encore plus préférentiellement au plus égale à 142 J/g d'échantillon.

4.  Composition édulcorante selon l'une quelconque des revendications 1 à 3, **caractérisée en ce que** son diamètre moyen volumique (moyenne arithmétique) D4, 3 est compris entre 250 et 350 $\mu$m, et de préférence entre 280 et 330 $\mu$m.

5.  Composition édulcorante selon l'une quelconque des revendications 1 à 4, **caractérisée en ce que** le sorbitol est constitué d'au moins 85% en poids de cristaux de forme y, de préférence d'au moins 90% en poids, ou plus préférentiellement d'au moins 95% en poids de cristaux de forme y.

6.  Composition édulcorante selon l'une quelconque des revendications 1 à 5, **caractérisée en ce qu'**elle présente une valeur d'hygroscopicité, déterminée par son évolution pondérale entre 0% et 60% d'humidité relative comprise entre 2,5 et 3,4%, de préférence entre 2,8 et 3,2%.

7.  Composition édulcorante selon l'une quelconque des revendications précédentes, **caractérisée en ce que** sa compressibilité est inférieure à 25%, de préférence comprise entre 7 et 22% et plus préférentiellement encore entre 10 et 20%.

8.  Utilisation de la composition édulcorante selon l'une quelconque des revendications 1 à 7, dans la fabrication de chewing-gums.

9.  Composition de chewing-gum contenant, les pourcentages étant indiqués en poids sec par rapport au poids sec total de la dite composition:

    - de 10% à 28%, préférentiellement de 15% à 25%, et encore plus préférentiellement 20% d'au moins une gomme de base,
    - de 20% à 70%, préférentiellement de 30% à 60 %, de la composition édulcorante selon l'une quelconque des revendications 1 à 7,
    - de 0,1 % à 5 %, préférentiellement de 0,5 % à 3 %, et encore plus préférentiellement de 1 à 1,8 % d'au moins un arôme.

10. Utilisation de la composition édulcorante selon l'une quelconque des revendications 1 à 7, dans la fabrication de comprimés à usage pharmaceutique ou alimentaire.

**Patentansprüche**

1.  Süßstoffzusammensetzung, **dadurch gekennzeichnet, dass** sie aufweist:

    - von 80 bis 95% des Trockengewichts an pulverförmigem kristallisiertem Sorbit,
    - eine Enthalpie von höchstens 150 J/g,
    - einen volumengemittelten Durchmesser zwischen 200 und 350 $\mu$m,
    - eine spezifische Oberfläche, bestimmt nach der BET-Methode, von kleiner als 0,6 m$^2$/g, bevorzugt zwischen 0,15 und 0,4 m$^2$/g und noch stärker bevorzugt zwischen 0,20 und 0,35 m$^2$/g.

**2.** Süßstoffzusammensetzung gemäß Anspruch 1, **dadurch gekennzeichnet, dass** sie zwischen 88% und 94,5% und stärker bevorzugt zwischen 90% und 94 % an pulverförmigem, kristallisiertem Sorbit aufweist.

**3.** Süßstoffzusammensetzung gemäß Anspruch 1 oder 2, **dadurch gekennzeichnet, dass** die Enthalpie höchstens 146 J/g der Probe und noch stärker bevorzugt höchstens 142 J/g der Probe ist.

**4.** Süßstoffzusammensetzung gemäß einem der Ansprüche 1 bis 3, **dadurch gekennzeichnet, dass** ihr volumengemittelter Durchmesser (arithmetisches Mittel) D4,3 zwischen 250 und 350 $\mu$m und bevorzugt zwischen 280 und 330 $\mu$m ist.

**5.** Süßstoffzusammensetzung gemäß einem der Ansprüche 1 bis 4, **dadurch gekennzeichnet, dass** der Sorbit aus mindestens 85 Gewichts-% an Kristallen der $\gamma$-Form, bevorzugt aus mindestens 90 Gewichts-% oder stärker bevorzugt aus mindestens 95 Gewichts-% an Kristallen der $\gamma$-Form besteht.

**6.** Süßstoffzusammensetzung gemäß einem der Ansprüche 1 bis 5, **dadurch gekennzeichnet, dass** sie einen Hygroskopizitätswert, bestimmt durch ihre Gewichtsveränderung zwischen 0% und 60% relativer Feuchtigkeit, zwischen 2,5 und 3,4%, bevorzugt zwischen 2,8 und 3,2% aufweist.

**7.** Süßstoffzusammensetzung gemäß einem der vorhergehenden Ansprüche, **dadurch gekennzeichnet, dass** ihre Kompressibilität kleiner als 25%, bevorzugt zwischen 7 und 22% und noch stärker bevorzugt zwischen 10 und 20% ist.

**8.** Verwendung der Süßstoffzusammensetzung gemäß einem der Ansprüche 1 bis 7 zur Herstellung von Kaugummis.

**9.** Kaugummi-Zusammensetzung enthaltend:

- 10% bis 28%, bevorzugt 15% bis 25% und noch stärker bevorzugt 20% wenigstens einer Gummi-Basis,
- 20% bis 70%, bevorzugt 30% bis 60% der Süßstoffzusammensetzung gemäß einem der Ansprüche 1 bis 7,
- 0,1 bis 5%, bevorzugt 0,5 bis 3% und noch stärker bevorzugt 1 bis 1,8% wenigstens eines Aromas,

wobei die Prozentangaben als Trockengewicht bezogen auf das Gesamttrockengewicht der Zusammensetzung angegeben sind.

**10.** Verwendung der Süßstoffzusammensetzung gemäß einem der Ansprüche 1 bis 7 zur Herstellung von Tabletten zur pharmazeutischen Verwendung oder zur Verwendung in der Ernährung.

**Claims**

**1.** A sweetening composition, **characterized in that** it has:

- from 80% to 95% by dry weight of crystalline pulverulent sorbitol,
- an enthalpy at most equal to 150 J/g,
- a volume mean diameter of between 200 and 350 $\mu$m,
- a specific surface area, determined according to the BET method, which is less than 0.6 m$^2$/g, preferably between 0.15 and 0.4 m$^2$/g, and even more preferentially between 0.20 and 0.35 m$^2$/g.

**2.** The sweetening composition as claimed in claim 1, **characterized in that** it has between 88% and 94.5%, and more preferentially between 90% and 94%, of crystalline pulverulent sorbitol.

**3.** The sweetening composition as claimed in claim 1 or 2, **characterized in that** the enthalpy is at most equal to 146 J/g of sample, and even more preferentially at most equal to 142 J/g of sample.

**4.** The hydrogenated sweetening composition as claimed in any one of claims 1 to 3, **characterized in that** its volume mean diameter (arithmetic mean) D4,3 is between 250 and 350 $\mu$m, and preferably between 280 and 330 $\mu$m.

**5.** The sweetening composition as claimed in any one of claims 1 to 4, **characterized in that** the sorbitol consists of at least 85% of crystals of $\gamma$ form, preferably of at least 90% by weight, or more preferentially of at least 95% by weight of crystals of $\gamma$ form.

6. The sweetening composition according to any one of claims 1 to 5, **characterized in that** it has a hygroscopicity value, determined by means of its weight change between 0% and 60% relative humidity, of between 2.5% and 3.4%, preferably between 2.8% and 3.2%.

7. The sweetening composition as claimed in any one of the preceding claims, **characterized in that** its compressibility is less than 25%, preferably between 7% and 22% and even more preferentially between 10% and 20%.

8. The use of the sweetening composition as claimed in any one of claims 1 to 7, in the production of chewing gums.

9. A chewing gum composition containing, with the percentages being given by dry weight relative to the total dry weight of said composition:

   - from 10% to 28%, preferentially from 15% to 25%, and even more preferentially 20% of at least one gum base,
   - from 20% to 70%, preferentially from 30% to 60%, of the sweetening composition as claimed in any one of claims 1 to 7,
   - from 0.1% to 5%, preferentially from 0.5% to 3%, and even more preferentially from 1% to 1.8% of at least one flavoring.

10. The use of the sweetening composition as claimed in any one of claims 1 to 7, in the production of tablets for pharmaceutical or food use.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 1506334
- FR 2622190
- EP 0347121 A
- EP 1008602 A
- FR 2202867

**Littérature non-brevet citée dans la description**

- **S. BRUNAUER et al.** BET Surface Area by Nitrogen Absorption. *Journal of American Chemical Society,* 1938, vol. 60, 309
- **P.W. ATKINS.** ATKINS et MORROW. 1986